# EUROPEAN PATENT SPECIFICATION

(11) **EP 0 406 978 B1**
(45) Date of publication and mention of the grant of the patent: **06.10.1993**
(21) Application number: 90201784.7
(22) Date of filing: 05.07.1990
(51) Int. Cl.: H02K 7/18, A01K 29/00, A61B 5/11, G01P 13/00

(54) **Motion sensor apparatus**
Bewegungssensor
Appareil capteur de mouvement

(30) Priority: 06.07.1989 NL 8901720
(43) Date of publication of application: 09.01.1991
(73) Proprietor: N.V. Nederlandsche Apparatenfabriek NEDAP, NL-7141 DE Groenlo (NL)
(72) Inventor: Hogen Esch, Johannes Harm Lukas, NL-7122 ZN Aalten (NL)
(74) Representative: Smulders, Theodorus A.H.J., Ir.

(56) References cited:
- CH-A- 352 969
- DE-A- 2 253 135
- FR-A- 2 482 826
- US-A- 4 051 397
- US-A- 4 618 861

## Description

This invention relates to a motion sensor apparatus for detecting the movements of objects with which the apparatus is connected, said apparatus being arranged to measure the accelerations to which the object is subjected and their intensity.

Known devices for detecting movements of people, animals and goods, as described, for example, in European patent 0087015, often use so-called mercury and reed switches. These switches indicate a certain movement by closing or interrupting an electrical contact, caused by such movement. The motion sensor apparatus in addition often includes means for identifying the object. The object can be identified in various ways. One way of accomplishing this is by means of a so-called responder in accordance with the principle of applicant's Netherlands patent 176404, in which use is made of an interrogation field. If the responder is present within this electromagnetic field, the responder withdraws a certain amount of energy from the field, which it uses for generating a unique code. One condition in this connection is that the interrogation field is sufficiently strong.

One disadvantage of known equipment is that they require an external power supply for their operation. Such external power supply should be connected to the motion sensor apparatus. It is especially when the apparatus is to be attached to objects moving in a relatively large area that problems may occur. In practice, the external power source mostly takes the form of a battery. A battery has the disadvantage of having a limited service life, the end of which cannot always be determined with accuracy. Moreover, when a battery is used, and also when a mercury switch is employed, there is the danger of pollution and possibly poisoning of the object and its surroundings.

Another disadvantage is that the mercury and reed switches only register the movements with reference to the number of times the electrical contact is opened and/or interrupted. What cannot be measured is the intensity of the movements.

Finally, mercury and reed switches are, in part, position-dependent. This drawback is in particular of importance for measuring movements of movable objects which can occupy different positions, such as animals.

Furthermore, US patent 4,618,861 discloses an oestrus detection system, which comprises a motion sensor providing electrical pulses. The pulses are counted by a counter, and the result of the counting is stored in a memory device of a wirelessly readable responder. The responder also includes an identification code. Furthermore, the pulses are supplied, after rectification, to a buffer capacitor which functions as a source of supply voltage. The pulses are generated by means of a hammer which strikes a piezoelectric element when the apparatus moves.

A drawback of the prior apparatus is that a piezoelectric element is quite vulnerable. Another drawback is that the prior motion sensor itself is incapable of accumulating energy. As a result, slow or low-intensity movements are not correctly detected. In fact, such movements either fail to produce a hammer stroke, or lead to an insufficient amount of electrical energy generated.

Document CH-A-352969 discloses such an accumulating energy device but used for an other application.

It is an object of the present invention to remedy the drawbacks and disadvantages outlined above, and, in general, to provide an effective and reliable sensor apparatus.

According to the present invention, there is provided a motion sensor apparatus for detecting movements of objects with which the apparatus is connected, said apparatus comprising a movable mass whose kinetic energy is converted by electromechanical means into electrical signals which are supplied to an electrical buffer device to provide a source of electric power, characterized in that the electromechanical means comprises an accumulator for accumulating the mechanical kinetic energy which in operation is provided by said movable mass, said accumulator releasing the kinetic energy accumulated when a given threshold value is exceeded.

A motion sensor apparatus according to the present invention can also register the intensity of the movements. When the intensity of movement is increased, the threshold value will be exceeded relatively more often, and consequently release the accumulated energy more often. The energy released can drive a generator. The electrical power provided by the generator can be stored in a buffer capacitor, for example, a so-called supercap capacitor. Each time when the generator supplies electrical power to the buffer capacitor, it is added to the power already stored. The power in the buffer capacitor can be continuously used for feeding the sensor apparatus.

The electrical power supplied by the generator is more than enough for feeding the sensor apparatus. If desired, the remaining power can be used for feeding other electrical measuring equipment for determining, for example, biometric parameters or feeding, for example, a responder circuit.

One application of the apparatus according to the present invention is in dairy farming, to determine when a cow is in oestrus using the relationship between this condition and the cow's activity or motility. By counting, for example, the number of times the kinetic energy is released by the accumulator, the present invention provides a simple way of determining increased mobility of a cow to indicate she is in oestrus.

Another application is the measurement of movements during the transportation of merchandise. When an apparatus according to the present invention is attached to valuable articles, movements during transport can be registered, and the likelihood of any damage during transport can be detected by means of the apparatus. For example, medical equipment may be very sensitive to exposure to major forces. In connection with the reliability of such equipment in operation, it may be interesting to have the possibility of establishing whether the equipment has indeed been subject to such forces during transport. In the case of other goods, such as fruit, it may be of importance to register movements, possibly in addition to other values, such as temperature, in connection with keeping characteristics.

As stated before, it is often necessary that an object can be identified if measurements are made with regard to it. An electronic responder used for this purpose can be so arranged that, in addition to the unique code of the object, the registered results of the measurements are passed to the transmitter/receiver through the interrogation field. The remaining energy in the buffer capacitor can also be used for providing the responder with the energy required for the functions referred to, so that the responder needs to withdraw relatively little, if any, energy from the interrogation field. In this way, the responder may, for example, be recognized from a larger distance, or a less strong interrogation field may be used.

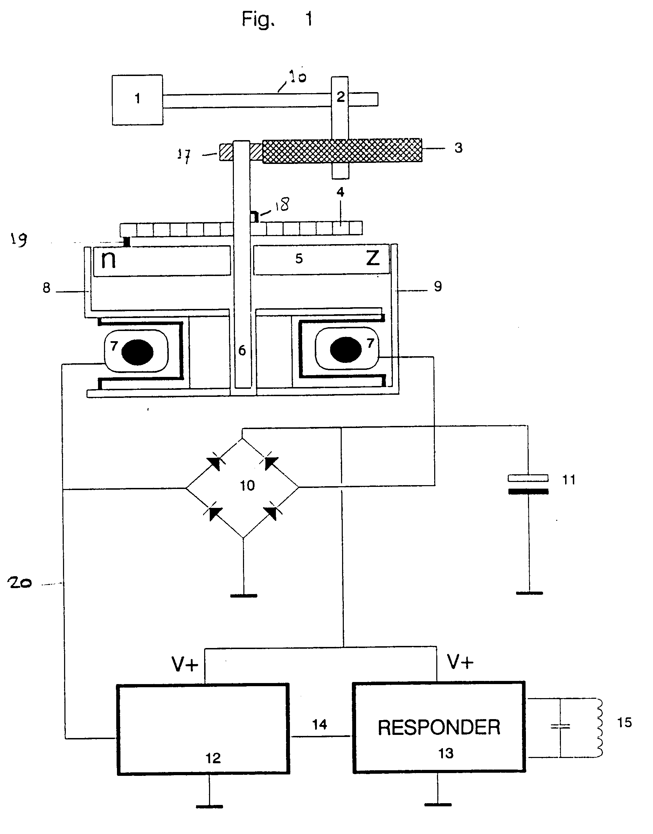

The invention will be described in more detail with reference to the accompanying drawing, which illustrates schematically one embodiment of an activity meter according to the present invention.

Referring to the accompanying drawing, there is shown a mass 1 which by way of an arm 16 and a shaft 2 is connected to a transmission 3,17. The mass 1 is capable of rotating in a plane transverse to the plane of drawing. In a given position of the meter, the mass rotates downwards about shaft 2 through gravity. The transmission 3,17, which in this example is a gear transmission, then moves a shaft 6. Owing to the movement of the mass, a spring 4 connected to shaft 6 is tensioned more and more. The connection of shaft 6 to spring 4 is shown schematically at 18. The weight of the mass 1 keeps the spring in the tensioned condition. The bearings of shafts 2 and 6 are not shown. A magnet 5 connected to spring 4 at 19, and magnetized, for example, to have 14 poles, is kept in position by the prevailing magnetic forces between magnet 5 and stator members 8 and 9. The magnetic field extends via the stator members 8 and 9, which are also, for example, 14-poled. Disposed between the two stator members 8 and 9 is a coil 7. When the force exerted by spring 4 on magnet 5 exceeds a given threshold value, and hence exceeds the magnetic force between magnet 5 and the stator members, the magnet is caused to rotate around shaft 6. During each revolution, the magnet is moved along the 14 poles of the two stator members. This creates a varying magnetic flux in coil 7 and generates an electric voltage in the coil.

It is observed that minor movements or accelerations will move mass 6, and hence increase the tension of the spring, but do not directly cause the threshold value to be exceeded. It is only when, after a number of such minor movements or accelerations the threshold value is exceeded that an electric voltage is generated by the magnet/coil combination, which functions as a dynamo. Accordingly, the spring acts as a mechanical accumulator for minor movements and accelerations.

By means of a rectifier, for example, a bridge rectifying circuit 10, the electrical pulses generated by the varying flux in the coil are rectified and stored as a charge in a supercap capacitor 11. As a consequence, the voltage across the capacitor rises slowly. This voltage can be used as a supply voltage for the activity measurement. The remaining voltage across capacitor 11 can be used to provide voltage to other equipment consuming electrical energy, such as a responder circuit 13. A part of the electrical pulses generated by the generator is withdrawn before the rectifying circuit 10 and, through a line 20, supplied to an activity measuring circuit 12. From the number of electrical pulses, via the activity measuring circuit, the activity can be determined by an external computer not shown. Circuit 12 may comprise a binary counter, for example. The electrical pulses registered by the activity measuring circuit are passed to a responder circuit 13 through a data line 14. Subsequently, in the presence of an interrogation field, the responder circuit transmits this data through antenna 15 to a transmitter/receiver not shown together with a code which is unique for this device.

After reading the above, various modifications will readily occur to one skilled in the art. Thus it would be possible to select the number of pulses per unit time or the number of times the magnet is brought into rotation as a motility measure. Furthermore, various types of transmission between mass 1 and shaft 6 are conceivable.

## Claims

1. A motion sensor apparatus for detecting movements of objects with which the apparatus is connected, said apparatus comprising a movable mass (1) whose kinetic energy is converted by electromechanical means into electrical signals which are supplied to an electrical buffer device (11) to provide a source of electric power, characterized in that the electromechanical means comprises an accumulator (4) for accumulating the mechanical kinetic energy which in operation is provided by said movable mass (1), said accumulator (4) releasing the kinetic energy accumulated when a given threshold value is exceeded.

2. Apparatus as claimed in claim 1, characterized in that the accumulator (4) for mechanical kinetic energy comprises a spring connected to a magnet (5), which magnet is kept in a quiescent state under the influence of magnetic forces.

3. Apparatus as claimed in claim 1 or 2, characterized in that the mechanical means comprises a transmission (3,17) between the movable mass (1) and a member (6) mounted for rotation and fixedly connected to one end of a coil spring (4), and a rotatable magnet (5) connected to the other end of said coil spring, there being provided at least one stator member (8,9) in the vicinity of said magnet (5), which stator member includes at least one coil (7) which provides an electrical signal when the magnet rotates.

4. Apparatus as claimed in claim 2 or 3, characterized in that the magnet (5) is a multi-poled magnet.

5. Apparatus as claimed in claim 2 or 3, characterized in that the stator member (8,9) is a multi-poled stator.

6. Apparatus as claimed in any one of claims 3-5, characterized in that the coil (7) is connected through a rectifier (10) to one or more circuits using electrical energy and to the buffer member (11), and that the coil is directly connected to an activity measuring circuit (12) to provide electrical pulses to the activity measuring circuit.

7. Apparatus as claimed in claim 6, characterized by an electronic responder circuit (13) provided with an identification code and connected to said activity measuring circuit, said electronic responder circuit generating both said identification code and the signal generated by the activity measuring circuit.

8. The use of a motion sensor apparatus as claimed in any one of claims 1-7 for the registration of movements of vulnerable goods during the transport thereof.

## Patentansprüche

1. Bewegungssensor zur Ermittlung der Bewegungen von Gegenständen, mit denen der Sensor verbunden ist, wobei der Sensor eine bewegliche Masse (1) umfaßt, deren Bewegungsenergie durch elektromechanische Mittel in elektrische Signale umgewandelt wird, die einem elektrischen Pufferorgan (11) zugeführt werden, um eine elektrische Stromquelle zu erzeugen, dadurch gekennzeichnet, daß die elektromechanischen Mittel einen Akkumulator (4) für durch die bewegliche Masse (1) im Betrieb erzeugte mechanische Bewegungsenergie umfassen, welcher Akkumulator (4) die gespeicherte Bewegungsenergie freigibt, wenn ein vorgegebener Schwellenwert überschritten wird.

2. Sensor nach Anspruch 1, dadurch gekennzeichnet, daß der Akkumulator (4) für mechanische Bewegungsenergie eine Feder umfaßt, die mit einem Magnet (5) verbunden ist, welcher Magnet unter dem Einfluß von Magnetkräften in Ruhelage gehalten wird.

3. Sensor nach Anspruch 1 oder 2, dadurch gekennzeichnet, daß die mechanischen Mittel eine Übertragung (3, 17) zwischen der beweglichen Masse (1) und einem fest mit dem einen Ende einer Spiralfeder (4) verbundenen, drehbar angeordneten Organ (6) umfassen, sowie einen mit dem anderen Ende der Spiralfeder verbundenen drehbaren Magnet (5), wobei nahe dem Magnet (5) mindestens ein Statororgan (8, 9) vorgesehen ist, das mit wenigstens einer Spule (7) versehen ist, die bei drehendem Magnet ein elektrisches Signal erzeugt.

4. Sensor nach Anspruch 2 oder 3, dadurch gekennzeichnet, daß der Magnet (5) ein mehrpoliger Magnet ist.

5. Sensor nach Anspruch 2 oder 3, dadurch gekennzeichnet, daß das Statororgan (8, 9) ein mehrpoliger Stator ist.

6. Sensor nach einem der Ansprüche 3 bis 5, dadurch gekennzeichnet, daß die Spule (7) über einen Gleichrichter (10) mit einem oder mehreren elektrische Energie verbrauchenden Kreisen und mit dem Pufferorgan (11) verbunden ist und daß die Spule unmittelbar mit einem Aktivitätsmeßkreis (12) verbunden ist, um den Aktivitätsmeßkreis mit elektrischen Impulsen zu versehen.

7. Sensor nach Anspruch 6, gekennzeichnet durch einen mit einem Identifizierungscode versehenen und mit dem Aktivitätsmeßkreis verbundenen elektronischen Responderkreis (13), der sowohl den Identifizierungscode als auch das durch den Aktivitätsmeßkreis erzeugte Signal erzeugt.

8. Verwendung eines Bewegungssensors nach einem der Ansprüche 1 bis 7 zur Registrierung der Bewegungen verletzlicher Waren während ihres Transports.

## Revendications

1. Appareil de détection de mouvement, destiné à détecter les mouvements d'objets auxquels l'appareil est relié, ledit appareil comprenant une masse mobile (1) dont l'énergie cinétique est transformée par des moyens électromécaniques en signaux électriques qui sont envoyés à un dispositif-tampon électrique (11) pour fournir une source de puissance électrique, caractérisé en ce que les moyens électromécaniques comprennent un accumulateur (4) servant à accumuler l'énergie cinétique mécanique qui en fonctionnement est fournie par ladite masse mobile (1), ledit accumulateur (4) libérant l'énergie cinétique accumulée lorsqu'une valeur de seuil donnée est dépassée.

2. Appareil selon la revendication 2, caractérisé en ce que l'accumulateur (4) d'énergie cinétique mécanique comprend un ressort relié à un aimant (5), cet aimant étant maintenu dans un état quiescent sous l'influence de forces magnétiques.

3. Appareil selon la revendication 1 ou 2, caractérisé en ce que les moyens mécaniques comprennent une transmission (3, 17) située entre la masse mobile (1) et un élément (6) monté à rotation et solidaire d'une extrémité d'un ressort spiralé (4), et un aimant rotatif (5) relié à l'autre extrémité dudit ressort spiralé, au moins un élément de stator (8, 9) étant prévu au voisinage dudit aimant (5), cet élément de stator comprenant au moins un bobinage (7) qui fournit un signal électrique lorsque l'aimant tourne.

4. Appareil selon la revendication 2 ou 3, caractérisé en ce que l'aimant (5) est un aimant multipolaire.

5. Appareil selon la revendication 2 ou 3, caractérisé en ce que l'élément de stator (8, 9) est un stator multipolaire.

6. Appareil selon l'une quelconque des revendications 3 à 5, caractérisé en ce que le bobinage (7) est relié par l'intermédiaire d'un redresseur (10) à un ou plusieurs circuits utilisant l'énergie électrique, ainsi qu'à l'élément tampon (11), et en ce que le bobinage est relié directement à un circuit de mesure d'activité (12) servant à créer des impulsions électriques envoyées au circuit de mesure d'activité.

7. Appareil selon la revendication 6, caractérisé en ce qu'un circuit électronique répondeur (13) est prévu, est doté d'un code d'identification et est relié audit circuit de mesure d'activité, ledit circuit électronique répondeur créant à la fois ledit code d'identification et le signal créé par le circuit de mesure d'activité.

8. Utilisation d'un appareil de détection de mouvement selon l'une quelconque des revendications 1 à 7 pour l'enregistrement des mouvements de marchandises fragiles au cours du transport de celles-ci.
